## Europäisches Patentamt
## European Patent Office
## Office européen des brevets

(19)

(11) Veröffentlichungsnummer : **0 217 840**
**B1**

(12) **EUROPÄISCHE PATENTSCHRIFT**

(45) Veröffentlichungstag der Patentschrift :
**13.12.89**

(51) Int. Cl.⁴ : **C 12 P 33/02, C 07 J 1/00**

(21) Anmeldenummer : **86901819.2**

(22) Anmeldetag : **26.03.86**

(86) Internationale Anmeldenummer :
**PCT/DE 86/00130**

(87) Internationale Veröffentlichungsnummer :
**WO/8605813 (09.10.86 Gazette 86/22)**

(54) VERFAHREN ZUR HERSTELLUNG VON 1-METHYL-1,4-ANDROSTADIEN-3,17-DION.

(30) Priorität : 03.04.85 DE 3512328

(43) Veröffentlichungstag der Anmeldung :
15.04.87 Patentblatt 87/16

(45) Bekanntmachung des Hinweises auf die Patenterteilung : 13.12.89 Patentblatt 89/50

(84) Benannte Vertragsstaaten :
AT BE CH DE FR GB IT LI LU NL SE

(56) Entgegenhaltungen :
EP–A– 0 129 500
Steroids. Vol. 11, No. 3, March 1968, Holden Day, San
Francisco (US) György Wix et al.:"Inhibition of steroid
nucleus degradation in mycobacterial transformation" see pages 401-413
Die Akte enthält technische Angaben, die nach dem
Eingang der Anmeldung eingereicht wurden und die
nicht in dieser Patentschrift enthalten sind.

(73) Patentinhaber : SCHERING AKTIENGESELLSCHAFT
Berlin und Bergkamen
Müllerstrasse 170/178 Postfach 65 03 11
D-1000 Berlin 65 (DE)

(72) Erfinder : PETZOLDT, Karl
Flachsweg 10
D-1000 Berlin 38 (DE)

## Beschreibung

Das erfindungsgemäße Verfahren wird unter den Bedingungen durchgeführt, die man üblicherweise bei der mikrobiologischen Dehydrierung von Substraten mit Mikroorganismen-Kulturen anwendet. So werden zunächst in allgemein üblichen Vorversuchen die günstigsten Fermentationsbedingungen, wie zum Beispiel Auswahl des günstigsten Nährmediums, des geeigneten Substratlösungs- oder Suspensionsmittels, der Substratkonzentration, der technischen Bedingungen wie Temperatur, Belüftung, pH-Wert und der optimalen Zeiten für Germination, Substratzugabe und Substratkontakt am Enzym des Mikroorganismus analytisch, insbesondere dünnschichtchromatographisch, ermittelt.

Dabei hat sich gezeigt, daß es zweckmäßig ist, Konzentrationen von etwa 100 bis 2 000 mg Substrat pro Liter Nährmedium einzusetzen. Der pH-Wert wird vorzugsweise auf einen Wert im Bereich von 5 bis 7 eingestellt. Die Züchtungstemperatur liegt im Bereich von 20-40 °C, vorzugsweise von 25-35 °C. Zur Belüftung werden vorzugsweise 0.5 bis 5 Liter Luft pro Minute pro Liter Kulturbrühe zugeführt. Die Umwandlung des Substrats wird zweckmäßigerweise durch Analysen von Probeextrakten verfolgt.

Die für diese Fermentation benötigten Mikroorganismen stehen der Fachwelt bei anerkannten Mikroorganismen-Sammlungen zur freien Verfügung. Geeignete Stämme sind Norcardien, wie Norcardia corallina ATCC 14 350, Mycobacterien, wie Mycobacterium rhodochvous ATCC 4 276 oder Fusarien, wie Fusarium solani ATCC 12 823.

Nach erfolgter Fermentation werden die Fermentationsprodukte in an sich bekannter Weise isoliert. Die Isolierung kann zum Beispiel in der Weise erfolgen, daß man die Fermentationsansätze mit einem nicht mit Wasser mischbaren organischen Lösungsmittel wie Äthylacetat, Butylacetat oder Methylisobutylketon, extrahiert, die Extrakte einengt und die so erhaltenen Rohprodukte gegebenenfalls durch Chromatographie und/oder Kristallisation reinigt.

Die nachfolgenden Ausführungsbeispiele dienen zur Erläuterung des erfindungsgemäßen Verfahrens.

Beispiel 1

Ein 2 l Erlenmeyerkolben, der 500 ml einer 30 Minuten bei 120 °C im Autoklaven sterilisierten Nährlösung, bestehend aus 0,5 % Dextrose Monohydrat, 0,5 % Hefeextrakt, 0,2 % Corn steep liquor und 0,1 % Pepton, pH 7,5, wird mit einer Schrägagarkultur des Stammes Norcardia corallina ATCC 14 350 beimpft und 48 Stunden auf einem Rotationsschüttler bei 30 °C geschüttelt.

Mit 250 ml dieser Anzucht skultur wird ein 20 l Vorfermenter beimpft, der mit 15 l eines 30 Min. bei 121 °C und 1,1 bar Überdruck sterilisierten Nährmediums der gleichen Zusammensetzung wie die Anzuchtskultur beschickt ist. Unter Zugabe von Silicon SH als Antischaummittel wird bei 29 °C und 0,7 bar Überdruck unter Belüftung (15 l/Min.) und Rühren (220 U/Min.) 24 Stunden germiniert.

Danach werden 0,9 l dieser Vorfermenterkultur unter sterilen Bedingungen entnommen und damit ein 20 l Hauptfermenter beimpft, der 14 l sterilisierte Nährlösung der gleichen Zusammensetzung wie die Vorfermenterkultur enthält. Nach einer Anwachsphase von 6 Stunden unter Vorfermenterbedingungen wird eine sterilfiltrierte Lösung von 4,5 g Metenolon in 60 ml Dimethylformamid zugegeben und weiter gerührt und belüftet.

Nach 56 Stunden Kontaktzeit ist die Umsetzung beendet. Die Kulturbrühe wird einmal mit der Hälfte, danach zweimal mit je 1/3 des Kulturvolumens mit Methylisobutylketon extrahiert, die Extrakte werden vereinigt und im Vakuum zur Trockne eingeengt. Den Rückstand nimmt man zur Entfernung des Antischaummittels in Methanol auf, filtriert vom ungelösten Antischaummittel durch ein doppeltes Faltenfilter ab, behandelt die Lösung mit A-Kohle und engt erneut zur Trockne ein. Der Rückstand wird nun aus Essigester umkristallisiert und im Vakuumtrockenschrank getrocknet. Man erhält 2,42 g (54,6 % d. Theorie) 1-Methyl-androstadiendion vom Schmelzpunkt 164-166 °C. Weitere 0,45 g (10 % d. Theorie) 1-Methyl-androstadiendion werden durch säulenchromatographische Reinigung der Kristallisationsmutterlauge erhalten.

Beispiel 2

Unter den Bedingungen des Beispiels 1 wird Metenolon durch Verwendung des Stammes Mycobacterium rhodochvous ATCC 4276 zu 1-Methyl-androstadiendion umgesetzt.

Beispiel 3

Unter den Bedingungen des Beispiels 1, jedoch unter Verwendung eines Nährmeiums bestehend aus 3 % Glucose, 1 % Corn steep liquor, 0,2 % NaNO$_3$, 0,1 % KH$_2$PO$_4$, 0,2 % K$_2$HPO$_4$, 0,05 % MgSO$_4 \cdot$ 7H$_2$O, 0,002 % FeSO$_4 \cdot$ 7H$_2$O und 0,05 % KCl, wird Metenolon mittels des Stammes Fusarium solani ATCC 12 823 zu 1-Methyl-androstadiendion umgesetzt.

## Patentansprüche

1. Verfahren zur Herstellung von 1-Methyl-1,4-androstadien-3,17-dion, dadurch gekennzeichnet, daß man 17β-Hydroxy-1β-methyl-5α-androst-1-en-3-on(Metenolon) mit einer Mikroorganismenkultur der Gattungen Norcardia, Mycobacterium oder Fusarium fermentiert.

2. Verfahren zur Herstellung von 1-Methyl-1,4-androstadien-3,17-dion gemäß Patentanspruch 1, dadurch gekennzeichnet, daß man 17β-Hydroxy-1β-

methyl-5α-androst-1-en-3-on mit einer Mikroorganismenkultur der Spezies Norcardia corallina ATCC 14 350, Mycobacterium rhodochvous ATCC 4 276 oder Fusarium solani ATCC 12 823 fermentiert.

## Claims

1. Process for the manufacture of 1-methyl-1,4-androstadiene-3,17-dione, characterised in that 17β-hydroxy-1β-methyl-5α-androst-1-en-3-one(metenolone) is fermented with a microorganism culture of the genera Norcardia, Mycobacterium or Fusarium.

2. Process for the manufacture of 1-methyl-1,4-androstadiene-3,17-dione according to patent claim 1, characterised in that 17β-hydroxy-1β-methyl-5α-androst-1-en-3-one is fermented with a microorganism culture of the species Norcardia corallina ATCC 14 350, Mycobacterium rhodochvous ATCC 4 276 or Fusarium solani ATCC 12 823.

## Revendications

1. Procédé pour préparer la méthyl-1 androstadiène-1,4 dione-3,17 caractérisé en ce qu'on fait fermenter l'hydroxy-17 méthyl-1 androsta-5 ène-1 one-3(méténolone) avec une culture de micro-organismes des genres Norcardia, Mycobactérium ou Fusarium.

2. Procédé pour préparer la méthyl-1 androstadiène-1,4 dione-3,17, selon la revendication 1, caractérisé en ce qu'on fait fermenter de l'hydroxy-17 méthyl-1 androsta-5 ène-1 one-3 avec une culture de micro-organismes de l'espèce Norcardia corallina ATCC 14 350, Mycobactérium rhodochvous ATCC 4 276 ou Fusarium solani ATCC 12 823.